(19) Europäisches Patentamt
European Patent Office
Office européen des brevets

(11) **EP 3 183 563 B1**

(12) **EUROPÄISCHE PATENTSCHRIFT**

(45) Veröffentlichungstag und Bekanntmachung des Hinweises auf die Patenterteilung:
**06.06.2018 Patentblatt 2018/23**

(21) Anmeldenummer: **15730756.2**

(22) Anmeldetag: **18.06.2015**

(51) Int Cl.:
**G01N 27/02** *(2006.01)*    **G01N 33/483** *(2006.01)*

(86) Internationale Anmeldenummer:
**PCT/EP2015/063705**

(87) Internationale Veröffentlichungsnummer:
**WO 2015/197461 (30.12.2015 Gazette 2015/52)**

(54) **MONITORING DER WIRKUNG VON SUBSTANZEN AUF IN VITRO GEWEBE**

MONITORING THE EFFECT OF SUBSTANCES ON IN VITRO TISSUE

SURVEILLANCE DE L'ACTION D'UNE SUBSTANCE DANS DES TISSUS IN VITRO

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.06.2014 DE 102014009537**

(43) Veröffentlichungstag der Anmeldung:
**28.06.2017 Patentblatt 2017/26**

(73) Patentinhaber: **Fraunhofer-Gesellschaft zur Förderung der angewandten Forschung e.V.
80686 München (DE)**

(72) Erfinder:
• **GROEBER, Florian
97072 Würzburg (DE)**
• **HANSMANN, Jan
97246 Eibelstadt (DE)**
• **WALLES, Heike
97082 Würzburg (DE)**

(74) Vertreter: **Schwahn, Hartmut et al
Gleiss Große Schrell und Partner mbB
Patentanwälte Rechtsanwälte
Leitzstraße 45
70469 Stuttgart (DE)**

(56) Entgegenhaltungen:
**US-A- 4 537 203**

• **WEGENER J ET AL: "Automated multi-well device to measure transepithelial electrical resistances under physiological conditions", BIOTECHNIQUES, INFORMA HEALTHCARE, US, Bd. 37, Nr. 4, 1. Januar 2004 (2004-01-01) , Seiten 590-597, XP008126784, ISSN: 0736-6205**
• **ERICK A WHITE ET AL: "On the correlation between single-frequency impedance measurements and human skin permeability to water", TOXICOLOGY IN VITRO, ELSEVIER SCIENCE, GB, Bd. 25, Nr. 8, 15. September 2011 (2011-09-15), Seiten 2095-2104, XP028110783, ISSN: 0887-2333, DOI: 10.1016/J.TIV.2011.09.011 [gefunden am 2011-09-22]**
• **GROEBER F ET AL: "Impedance Spectroscopy for the Non-Destructive Evaluation ofIn VitroEpidermal Models", PHARMACEUTICAL RESEARCH, SPRINGER NEW YORK LLC, US, Bd. 32, Nr. 5, 3. Dezember 2014 (2014-12-03), Seiten 1845-1854, XP035444576, ISSN: 0724-8741, DOI: 10.1007/S11095-014-1580-3 [gefunden am 2014-12-03]**

## Beschreibung

Die Erfindung betrifft Verfahren und Mittel zur zerstörungsfreien Charakterisierung biologischer Gewebe *in vitro,* dabei besonders die Bestimmung der Wirkung von Substanzen auf das biologische Gewebe oder die Bestimmung von dessen Reife oder Differenzierungsgrades, und zwar anhand nicht invasiv und insbesondere wiederkehrend messbarer elektrischer Kenngrößen. Diese Kenngrößen sind durch ein neuartiges Verfahren aus der gemessenen elektrischen Impedanz des biologischen Gewebes ermittelbar.

In der biologischen Grundlagenforschung, aber insbesondere in der angewandten biomedizinischen Forschung stellen *in vitro* kultivierte biologische Gewebe, besonders auch mehrschichtige Gewebe, vor allem *de novo* aus isolierten Einzelzellen rekonstituierte so genannte Gewebeäquivalente wichtige Werkzeuge zur Untersuchung biologischer, das heißt besonders physiologischer oder zellulärer, Wirkungen von Substanzen und Wirkstoffen dar. Ein besonderes Einsatzgebiet ist die Ermittlung irritativer Wirkungen von Substanzen auf die Haut. Die Bereitstellung von und die Bestimmung der Substanzwirkung anhand von *in vitro* Gewebeäquivalenten ist eine attraktive Alternative gegenüber den bisher vorgeschriebenen Versuchen am lebenden Tier sowohl in der medizinischen Forschung, als auch in der kosmetischen Produktion.

Neben den aus dem tierischen oder menschlichen Körper direkt explantierten Geweben oder Biopsaten, welche meist zu medizinischen Forschungszwecken in Bioreaktoren kultiviert werden können, sind besonders für Testreihen besonders die aus isolierten Einzelzellen und/oder Zelllinien mehrschichtig *de novo* aufbaubaren Gewebeäquivalente anwendbar. Dabei können diese *in vitro* Gewebeäquivalente auch auf die konkrete Test- und Fragestellung in ihrer Zusammensetzung und/oder ihrem zellulären Aufbau angepasst werden. Problematisch ist dabei, diese *in vitro* Gewebeäquivalente (z. B. In-vitro-Hautäquivalente) in großer Zahl und in gleichbleibender Qualität, insbesondere in Struktur, Reifungs- oder Differenzierungsgrad, reproduzierbar bereitzustellen. Es ist wünschenswert, eine laufende Kontrolle und/oder eine Endkontrolle zur Verfügung zu haben, welche eine zuverlässige Aussage über die Qualität der in Serie produzierten Gewebeäquivalenten zulassen.

Daneben sind bekannte nicht invasive Verfahren zur Bestimmung, insbesondere der Quantifizierung einer Substanzwirkung in, insbesondere mehrschichtigen, biologischen *in vitro* Geweben nicht sensitiv genug, um vorallem irritative oder gar sub-irritative Vorgänge an en *in vitro* Geweben sicher detektieren und insbesondere auch quantifizieren zu können. Es ist wünschenswert, verbesserte, vor allem empfindlichere und zeitlich höher aufgelöste nicht invasive Kontrollverfahren zur Identifizierung und/oder Quantifizierung von Substanzwirkungen und deren Wirkungskinetik zur Verfügung zu haben, welche genauere und weitergehende Aussagen erlauben.

Der Erfindung liegt zum einen das Problem zugrunde, ein verbessertes zerstörungsfreies Verfahren zur Bestimmung von Vorgängen in *in vitro* kultivierten biologischen Geweben bereitzustellen. Damit verbunden ist das technische Problem, der Bereitstellung von Verfahren und Mitteln zur Charakterisierung eines *in vitro* Gewebes anhand von Kenngrößen. In diesem Zusammenhang steht auch das technische Problem, Verfahren und Mittel bereitzustellen, die besonders sensitiv die biologische Wirkung von Substanzen auf *in vitro* kultiviertes mehrschichtiges biologisches Gewebe nachweisen können. Damit verbunden ist auch das technische Problem, Verfahren und Mittel bereitzustellen, die eine nichtinvasive, das heißt besonders laufende Überwachung oder eine zerstörungsfreie Prozessendkontrolle des Zustandes von standardisierten, für Testzwecke einzusetzenden biologischen Geweben *in vitro,* insbesondere *in vitro* Gewebeäquivalenten, ermöglichen. Dabei soll insbesondere auch der Grad der zellulären Differenzierung beziehungsweise Reifegrad des biologischen Gewebes leicht und zuverlässig bestimmbar sein. Daneben soll eine hohe zeitliche Auflösung, das heißt hohe Abtastraten, bei der Messung erreicht werden.

Die vorliegende Erfindung löst das zugrunde liegende technische Problem vollständig durch die Bereitstellung eines Verfahrens zur Charakterisierung eins biologischen Gewebes gemäß Anspruch 1 sowie von Mitteln die spezifisch ausgebildet sind, das erfindungsgemäße Verfahren durchführen zu können.

Gegenstand der Erfindung ist besonders ein Verfahren zur Charakterisierung eines, insbesondere mehrschichtigen, biologischen Gewebes *in vitro,* enthaltend die Schritte:

a) Messen der elektrischen Impedanz des biologischen Gewebes in Abhängigkeit von der Frequenz, wobei ein Impedanzspektrum $Z(\omega)$ erhalten wird, und

b) iteratives Anpassen von *n* Modellspektren $Z_m(\omega)n$ an das gemessene Impedanzspektrum $Z(\omega)$, wobei jedes Modellspektrum $Z_m(\omega)n$ jeweils durch eine Formel bestimmt ist, die ausgewählt ist aus der Formelschar mit *n* Formeln gemäß Formel (1):

$$Z_m(\omega)n = R_S + Z_{CPE}(\omega) + \sum Z_{Cell}(\omega)i \text{ , für } i = 1 \text{ bis } n \qquad (1)$$

wobei für jedes *i*

$Z_{Cell}(\omega)i$ die spezifische Impedanz einer elektrisch wirksamen Schicht *i* in dem Gewebe darstellt, wobei jede elektrisch wirksame Schicht *i* jeweils einem Netzwerk in Form einer Parallelschaltung der ohmschen Komponente $R_{Cell}i$ dieser Schicht und der realen kapazitiven Komponente $C_{Cell}(\omega)(Ni)i$ dieser Schicht gemäß Formel (2) entspricht:

$$ZCell(\omega)i = R_{Cell}i \ \text{II} \ C_{Cell}(\omega)(Ni)i \hspace{3cm} (2)$$

und wobei
N*i* die Idealität dieser realen kapazitiven Komponente $C_{Cell}(\omega)(Ni')i$ ist, und
c) Bestimmen für jedes der *n* Modellspektren $Z_m(\omega)n$ das für jedes *n* jeweils optimal an das gemessene Impedanzspektrum $Z(\omega)$ angepasste Modellspektrum,
d) Ermitteln mindestens einer Kenngröße, ausgewählt aus ohmscher Komponente $R_{Cell}$ und realer kapazitiver Komponente $C_{Cell}(\omega)(N)$ jedes der angepassten *n* Modellspektren als die das biologische Gewebe charakterisierende Kenngröße.

Dabei werden für jedes *n* die Modellspektren $Z_m(\omega)n$ jeweils durch automatische Variation von jeweils mindestens einer der impedanzbestimmenden Komponenten $R_{Cell}i$ und $C_{Cell}(\omega)(Ni)i$ jeder elektrisch wirksamen Schicht *i* an das in Schritt a) gemessene Impedanzspektrum $Z(\omega)$ angenähert. Bevorzugt ist dabei vorgesehen, dass die interative Anpassung in Schritt b) besonders nach der Methode der kleinsten Differenzenquadrate automatisch erfolgt. In Schritt c) ist für jedes *n* das jeweils optimal angepasste Modell dadurch gekennzeichnet ist, dass es die jeweils kleinsten Residuen gegenüber dem gemessenen Impedanzspektrum aufweist.

Es werden so also für jedes der *n* Modelle jeweils ein optimal angepasstes, das gemessene Impedanzspektrum optimal repräsentierende Modellspektrum gefunden. Es ist in einer Variante vorgesehen, dass zu Beginn oder am Ende der automatischen Analyse zur Bestimmung der Modellspektren und der daraus erhältlichen Kennwerte, die für das jeweils zu untersuchende, insbesondere mehrschichtige Gewebe zweckmäßige Zahl an elektrisch wirksamen Schichten durch Auswahl der Zahl *n* vorzugeben. Werden zum Beispiel aufgrund zellbiologischer Erkenntnisse, histologischer Befunde und/oder früherer Messungen in einem mehrschichtigen Gewebe zwei elektrisch wirksame Schichten erwartet, kann die Zahl *n* = 2 gewählt werden, um die Analyse auf ein Modell mit genau zwei Schichten *i* zu beschränken. Zeigt sich beispielsweise in einem mehrschichtigen Gewebe eine einzige prominente Schicht mit prominenten elektrischen Eigenschaften bezüglich der Impedanz des gesamten Gewebes, kann die Zahl *n* = 1 gewählt werden, um die Analyse auf ein Modell mit genau eine Schicht *i* zu beschränken. Bevorzugt ist daher ein sozusagen kursorisches Verfahren wobei *n* = 1 oder *n* = 2 gewählt wird. Bei diesem kursorischen Ansatz der erfindungsgemäßen Analyse kann mit den so gefundenen Kenngrößen in vielen Fällen eine hinreichend genaue und sensitive Charakterisierung einer Substanzwirkung auf biologische Gewebe erfolgen, wobei gleichzeitig der Aufwand an Ressourcen der elektronischen Datenverarbeitung klein gehalten werden kann.

In einer dazu alternativen bevorzugten Variante ist vorgesehen, dass in Schritt c) unter den *n* gefundenen optimal angepassten Modellen $Z_m(\omega)n$, dasjenige als das bestangepasste Modell $Z_m(\omega)$ ausgewählt wird, welches über alle *n* Modelle betrachtet, die kleinsten Abweichungen zu dem gemessenen Impedanzspektrum $Z(\omega)$ aufweist und in Schritt d) die mindestens eine Kenngröße aus diesem bestangepassten Modellspektrum $Z_m(\omega)$ ermittelt wird. Durch diese automatisierte Anpassung der Modelle auch in Bezug auf die Anzahl der im Modell enthaltenen elektrisch wirksamen Schichten *i* können automatisch diejenigen Kenngrößen ermittelt werden, welche das biologische Gewebe am besten charakterisieren, und gleichzeitig eine Aussage über die aktuell vorhandene Zahl an elektrisch wirksamen Schichten in dem biologischen Gewebe getroffen werden.

Demgemäß sieht die Erfindung bevorzugt vor, dass in Schritt d) für jede elektrisch wirksame Schicht *i* des gefundenen bestangepassten Modellspektrums mindestens eine Kenngröße, ausgewählt aus ohmscher Komponente $R_{Cell}i$ und kapazitiver Komponente $C_{Cell}(\omega)(Ni')i$ als eine genau diese elektrisch wirksame Schicht *i* des biologischen Gewebes charakterisierende Kenngröße getrennt ermittelt wird.

Demgemäß ist bevorzugt weiter vorgesehen, dass in der Formel $Z_m(\omega)n$ des gefundenen bestangepassten Modellspektrums *n* die Zahl der in dem biologischen Gewebe aktuell vorherrschenden elektrisch wirksamen Schichten anzeigt.

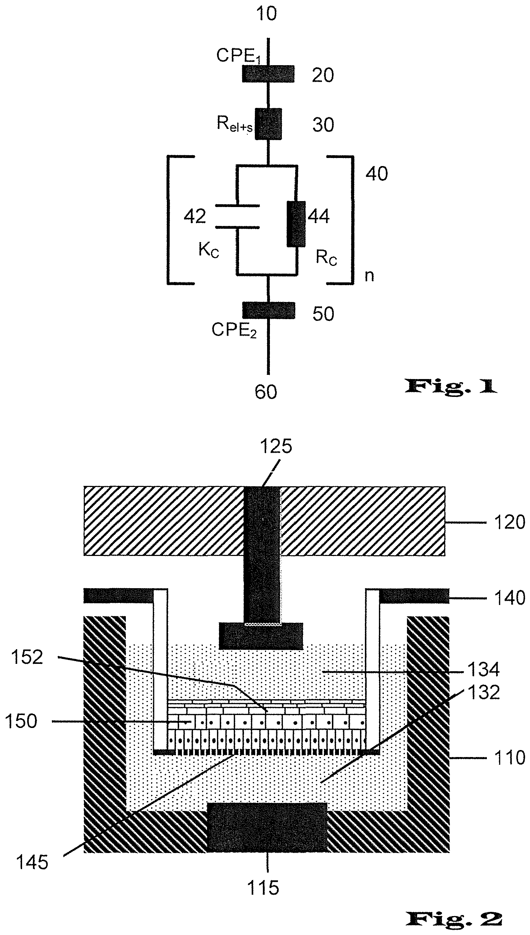

Das erfindungsgemäße Verfahren sieht also besonders vor, aus einem an sich bekannter Weise von dem biologischen Gewebe nicht invasiv ermittelten elektrischen Impedanzspektrum durch Modellierung bestimmte elektrische Kennwerte zu extrahieren, die robuste Aussagen über dem physiologischen Zustand des biologischen Gewebes erlauben. Zur Modellierung wird ein neuartiges Modell der Verschaltung der elektrischen Hauptkomponenten in einem biologischen Gewebe eingesetzt. Es versteht sich, dass das Gewebe im Wesentlichen auf einer Membran oder ähnlichem kultiviert oder fixiert ist und eine Bioreaktorkammer in einer obere (apikale) und eine untere (basale) Kammer teilt. Die elektrische Impedanz wird entlang des Vektors apikal zu basal, das heißt senkrecht zur Ebene des biologischen Gewebes gemessen. Die Elektroden zur Impedanzmessung befinden sich in an sich bekannter Weise zum einen in der apikalen Kammer des Bioreaktors und zum anderen in der basalen Kammer des Bioreaktors. Entsprechend erstreckt sich das Ersatzschaltbild des biologischen Gewebes entlang dieser beiden Bezugspunkte. Das der Erfindung zugrundeliegende Ersatzschaltbild eines Gewebes ist in Figur 1 dargestellt. Es besteht im Wesentlichen aus einem zusammengefassten und im wesentlichen frequenzunabhängigen Serienwiderstand $R_s$ in Reihe geschaltet mit einem zusammengefassten sogenannten konstanten Phasenelement, welches frequenzabhängig ist, jedoch im Wesentlichen unabhängig von den biologischen Vorgängen in den biologischen Gewebe ist und damit nicht zu den erfindungsgemäß ermittelten Kennwerten beiträgt. Weiter in Reihe geschaltet ist mindestens ein Netzwerk bestehend aus einer Parallelschaltung einer Ohm'schen Komponente einer elektrisch relevanten Schicht in dem biologischen Gewebe und einer kapazitiven Komponente dieser Schicht. Bei der kapazitiven Komponente wird in Abweichung von den Lehren des Standes der Technik das Modell eines sogenannten realen Kondensators verwendet. Ein realer Kondensator zeichnet sich durch eine Idealität **N** < 1 aus, welche, ohne an eine Theorie gebunden sein zu wollen, der "nach Rauigkeit" des Kondensators Rechnung trägt. Demgemäß berechnet sich der Impedanzanteil dieses Netzwerks einer elektrisch wirksamen Schicht nach Formel (2):

$$ZCell(\omega)i = R_{Cell}i \; \text{II} \; C_{Cell}(\omega)(Ni)i \tag{2}$$

Gemäß der Erfindung sieht das Ersatzschaltbild nun zusätzlich die Möglichkeit vor, dass zwar mindestens eins dieser Netzwerke, aber auch mehrere Netzwerke (**n** > 1) in Reihe geschaltet sein können. Gemäß der Erfindung wird eine elektrisch wirksame Schicht in die biologischen Gewebe durch ein solches Netzwerk charakterisiert. Demgemäß rechnet sich die Gesamtimpedanz des elektrischen Ersatzschaltbilds, worauf die vorliegende Erfindung beruht, je nach Anzahl **n** der vorhandenen elektrisch wirksamen Schichten in dem mehrschichtigen biologischen Gewebe nach:

$$Z_m(\omega)n = R_S + Z_{CPE}(\omega) + \sum Z_{Cell}(\omega)i \text{ , für } i = 1 \text{ bis } n \tag{1}$$

Zur Analyse des gemessenen Impedanzspektrums wird nun in an sich bekannter Weise der sich aus dem gewählten elektrischen Ersatzschaltbild ergebende modellierte Verlauf der elektrischen Impedanz (Modellspektrum) an das gemessene Impedanzspektrum angepasst, das heißt gefittet. Als erfindungsgemäße charakteristische Kenngrößen werden zumindest die kapazitive Komponente und/oder die Ohm'sche Komponente einer elektrisch wirksamen Schicht entsprechenden parallelen Netzwerks in dem elektrischen Ersatzschaltbild ermittelt. Dabei ist bevorzugt vorgesehen, dass für jede elektrisch wirksame Schicht (**n** > 2) die wirksame "Schicht" übereinstimmend mit einer bestimmten Zellschicht. In anderen Fällen wird die elektrisch wirksame Schicht durch andere gegebenenfalls nicht zelluläre Strukturen in dem biologischen Gewebe gebildet. Beispiele dafür sind Hornschichten, Bindegewebsschichten, Gefäßschichten oder prominent ausgeprägte Basalmembranen. Auch eine in dem biologischen Gewebe vorhandene ausgeprägte extrazelluläre Matrix kann eine solche elektrisch wirksame Schicht bilden. Die Erfindung sieht also vor, ein Ersatzschaltbild zur Analyse von Impedanzspektren heranzuziehen, welche von der Idee einer sogenannten elektrisch wirksamen Schicht Gebrauch machen. Als konkretes Beispiel dient beispielsweise eine explantierte *in vitro* kultivierte Hornhaut des Wirbeltierauges (Cornea), welches im Wesentlichen aus einer Epithelschicht und einer darunterliegenden Endothelschicht besteht. Sowohl die Epithelschicht als auch die Endothelschicht bildet jeweils eine elektrische wirksame Schicht, die in dem erfindungsgemäßen Ersatzschaltbild unterscheidbar ist.

Gegenstand der Erfindung ist also ein Verfahren zur Charakterisierung eines biologischen Gewebes *in vitro,* enthaltend die Schritte: a) Messen der elektrischen Impedanz des biologischen Gewebes in Abhängigkeit von der Frequenz, wobei ein Impedanzspektrum $Z(\omega)$ erhalten wird, b) Anpassen an das gemessene Impedanzspektrum $Z(\omega)$, wobei ein Modellspektrum $Zm(\omega)n$ durch eine Formel bestimmt ist, die ausgewählt ist aus der Formelschar: $Z_m(\omega)n = R_S + Z_{CPE}(\omega) + \text{Summe } (Z_{Cell}(\omega)i)$ für $i = 1$ bis $n,$ wobei $n$ $Z_{Cell}(\omega)i$ die Impedanz einer elektrisch wirksamen Schicht $i$ in dem Gewebe ist, die einer Parallelschaltung der ohmschen Komponente $R_{Cell}i$ dieser Schicht und der realen kapazitiven Komponente $C_{Cell}(\omega)(Ni)i$ dieser Schicht entspricht: $Z_{Cell}(\omega)i = R_{Cell}i$ parallel $C_{Cell}(\omega)(Ni)i$ und wobei N$i$ die Idealität dieser realen kapazitiven Komponente $C_{Cell}(\omega)(Ni)i$ ist und stets kleiner als 1 ist, wobei für jedes n die Modellspektren $Zm(w)n$ jeweils durch Variation von jeweils mindestens einer der impedanzbestimmenden Komponenten Reell i und kapazitiver Komponente CCell ($\omega$) (Ni)i jeder elektrisch wirksamen Schicht i an das in Schritt a) gemessene Impedanzspektrum $Z(w)$ angenähert werden; c) Bestimmen aus für jedes Modellspektrum $Z_m(\omega)n$ das jeweils optimal an das gemessene Impedanzspektrum $Z(\omega)$ angepasste Modellspektrum, d) Ermitteln mindestens einer Kenngröße, ausgewählt aus ohmscher Komponente $R_{Cell}$, kapazitiver Komponente jedes der angepassten Modellspektren als die das biologische Gewebe charakterisierende Kenngröße, wobei bevorzugt in Schritt b) für jedes $n$ die Modellspektren $Z_m(\omega)n$ jeweils durch Variation von jeweils mindestens einer der impedanzbestimmenden Komponenten $R_{Cell}i$ und kapazitiver Komponente $C_{Cell}(\omega)(Ni)i$ jeder elektrisch wirksamen Schicht $i$ an das in Schritt a) gemessene Impedanzspektrum $Z(\omega)$ angenähert werden, und/oder wobei bevorzugt die interaktive Anpassung in Schritt b) nach der Methode der kleinsten Differenzenquadrate erfolgt und in Schritt c) für jedes $n$ das jeweils optimal angepasste Modell dadurch gekennzeichnet ist, dass es die jeweils kleinsten Residuen gegenüber dem gemessenen Impedanzspektrum aufweist.

Die Erfindung sieht in diesem Zusammenhang besonders vor, gezielt die elektrischen Kenngrößen einer spezifischen elektrisch wirksamen Schicht, im Falle der Cornea der Epithelschicht oder der Endothelschicht, getrennt zu analysieren. Ein anderes konkretes Beispiel ist die Epithelschicht der Haut oder eines Hautäquivalents, welche aus einer Hornschicht (Stratum corneum) und darunter liegenden Epithelzellen besteht. Eine erste elektrisch wirksame Schicht wird durch diese nicht vitale Hornschicht gebildet, eine zweite elektrische wirksame Schicht durch die Epithelzellschicht. In einem jungen Hautgewebe oder Hautäquivalent ist diese Hornschicht vergleichsweise noch dünn ausgebildet und stellt eine gegenüber der Epithelzellschicht bezüglich der Größe der elektrischen Kenngrößen vergleichbare elektrische wirksame Schicht dar. Demgegenüber weist die reife Haut beziehungsweise ein gereiftes Hautäquivalent eine dickere Hornschicht auf, welche bezüglich der Größe der elektrischen Kenngrößen gegenüber der Epithelschicht in den Vordergrund tritt. In einer erfindungsgemäßen Modellierung wäre junges, unreifes Hautepithel am besten anhand eines Ersatzschaltbildes, welches zwei, insbesondere gleichgewichtete elektrisch wirksame Schichten aufweist ($n$ = 2) repräsentiert, wohingegen reifes Hautepithel mit dicker Hornschicht durch ein Ersatzschaltbild mit einer einzigen prominenten elektrisch wirksamen Schicht ($n$ = 1) repräsentiert ist.

Im Zusammenhang mit der vorliegenden Erfindung wird unter einem "mehrschichtigen" biologischen Gewebe nicht notwendigerweise ein Gewebe verstanden, dass zwei, anatomisch unterscheidbare vor allem voneinander verschiedene vitale Zellschichten aufweist, beispielsweise Keratinozyten und Fibroblasten. Es werden hierunter auch Gewebe verstanden, bei denen Schichten vitaler Zellen und nicht vitale Schichten, beispielsweise eine Hornschicht (*Stratum corneum*) vorhanden sind. Weiterhin versteht sich, dass eine anatomisch oder histologisch definierbare Schicht eines biologischen Gewebes nicht notwendigerweise jeweils mit einer einzigen sogenannten elektrisch wirksamen Schicht gemäß des erfindungsgemäßen Ersatzschaltbilds korrespondiert. Mehrere histologisch unterscheidbare Schichten eines mehrschichtigen Gewebes können also auch durch eine einzige elektrisch wirksame Schicht repräsentiert sein.

In besonderer Ausgestaltung erlaubte die Erfindung den akuten Einfluss von Wirkstoffen, aber auch Veränderungen im Rahmen der Reifung und Differenzierung des Gewebes auf bestimmte Schichten zu begrenzen oder dort gezielt zu untersuchen.

Bevorzugt ist das zu untersuchende biologische Gewebe ausgewählt aus: *de novo* aus isolierten Zellen des menschlichen oder tierischen Körpers und/oder aus Zelllinien rekonstituiertem Gewebeäquivalent und explantiertem *ex vivo* Gewebe (Biopsat) des menschlichen oder tierischen Körpers. in einer besonderen Ausgestaltung der Erfindung ist das biologische Gewebe ein so genanntes In-vitro-Hautäquivalent, wie dies besonders in der DE 10 062 623 A1 und der DE 10 2010 023 156 A1 beschrieben wurde, deren diesbezüglicher Offenbarungsgehalt hiermit in die Offenbarung der Erfindung mit eingeschlossen sein soll.

Die Erfindung ist aber nicht auf die Anwendung bei Hautgeweben beschränkt. Andere bevorzugt einsetzbare *ex vivo* Gewebe und *in vitro* Gewebeäquivalente sind ausgewählt aus: Darmepithel, Netzhaut, Cornea, Nierengewebe, Lebergewebe und anderen.

Eine spezifische Anwendung dieser Erfindung ist die Charakterisierung, Klassifizierung und insbesondere Quantifizierung subirritativer, irritativer und/oder korrosiver Wirkungen von Substanzen auf das biologische Gewebe. Eine spezifische Anwendung davon ist die Bestimmung korrosiver und/oder irritativer Wirkungen von Testsubstanzen auf die Haut welche von in Form von In-vitro-Hautäquivalenten mittels des erfindungsgemäßen Verfahrens und/oder der erfindungsgemäßen Mittel zuverlässig und sensitiv erfolgen kann. Vor allem erlaubt die Erfindung eine genauere Quantifizierung und Klassifizierung der Substanzwirkungen als dies mit bekannten Mess- und Analyseverfahren bisher möglich war. Es kann vorteilhafterweise eine korrosive Wirkung, das heißt eine irreversible Schädigung des Gewebes, von einer irritativen Wirkung, das heißt einer reversiblen Beeinträchtigung des Gewebes, und von einer sub-irritativen Wirkung, das heißt einer physiologischen Wirkung des Gewebes ohne Schädigung, unterschieden werden. Besonders im Zusammenhang mit der Untersuchung von Substanzwirkungen auf die Haut in Form von In-vitro-Hautäquivalenten sind solche Klassifizierungen zweckmäßig, wobei insbesondere der sicheren Detektion der sub-irritativen Wirkung einer Substanz, die allgemein als "burn", "itch" oder "sting" bezeichnet wird, große Bedeutung zukommt.

Im Zusammenhang mit der Erfindung hat sich beispielsweise herausgestellt, dass eine sub-irritative Wirkung, die mit den vorgeschriebenen Sensationen einhergehen, überwiegend durch Vorgänge in nicht vitalen Schichten, hier: Hornschicht der Epithelzellschicht, einhergehen. Beispielsweise wirken sub-irritativ wirkende Lösungsmittel wie 2-Propanol überwiegend in der nicht vitalen Hornschicht, indem sie dort beispielsweise die Löslichkeit von barrierebildenden Komponenten erhöhten, wodurch die Hornschicht durchlässiger wird. Dies lässt sich mittels des erfindungsgemäßen Verfahrens detektieren und vorteilhafterweise auch quantifizieren. Das heißt, die beobachtbaren Änderungen elektrischer Kenngrößen in dem Gewebe sind nicht notwendigerweise auf biologische Vorgänge innerhalb von Schichten vitaler Zellen zurückzuführen, sondern können auch durch rein chemisch physikalische Vorgänge in nicht vitalen Schichten begründet sein.

Im Zusammenhang mit der Untersuchung von Hautgeweben, besonders in Form von In-vitro-Hautäquivalenten, hat sich überraschend gezeigt, dass eine hoch irritative oder korrosive Wirkung einer Substanz mit einer starken Herabsetzung der Kenngröße "Ohm'scher Widerstand" in der elektrisch relevanten Schicht und mit einem starken Anstieg der Kenngröße "reale Kapazität" in dieser Schicht einhergeht. Im Gegensatz dazu bewirkt eine schwach irritative Wirkung zwar ebenfalls eine starke Herabsetzung des Ohm'schen Widerstands, die reale Kapazität ist aber dabei weit weniger verändert, und die Kenngrößen gehen im Zeitverlauf nach Ende der Substanzeinwirkung wieder weitgehend auf ihre ursprünglichen Werte zurück, was die Reversibilität der Schädigung charakterisiert. Im Falle der sub-irritativen Wirkung einer Substanz tritt eine beobachtbare Änderung nur oder weitgehend ausschließlich in dem Ohm'schen Widerstand auf. Diese sind außerdem nur sehr kurzzeitig nach dem ersten Inkontaktbringen und danach vollständig reversibel. Aufgrund der Robustheit der Analyseergebnisse lassen sich mit dem erfindungsgemäßen Verfahren statistisch belastbare Aussagen zur Klassifizierung der Substanzwirkung sowie auch zu Dosis/Wirkung-Zusammenhängen bereits bei geringen Stichprobenzahlen realisieren.

Die Erfindung basiert auf der Analyse von Daten einer elektrischen Impedanzmessung. Die Impedanzmessung an dem Gewebe kann in an sich bekannter Weise erfolgen. Bevorzugt erfolgt in Schritt a) die Messung des Impedanzspektrums des biologischen Gewebes durch Einprägen eines Wechselstroms mit wechselnden Frequenzanteilen, bevorzugt im Bereich von 1 Hz bis 100 kHz, mehr bevorzugt von 20 Hz bis 20 kHz, und Messen der frequenzabhängigen Wechselspannung die über die Gewebeschichten hinweg abfällt. Bevorzugt wird ein Wechselstrom von etwa 2 bis 5 mA, bevorzugt maximal etwa 3 mA eingeprägt und der resultierende frequenzabhängige Spannungsabfall gemessen.

In einer alternativen Variante der Impedanzmessung erfolgt in Schritt a) die Messung des Impedanzspektrums des biologischen Gewebes durch Anlegen einer Wechselspannung mit wechselnden Frequenzanteilen, bevorzugt im Bereich von 1 Hz bis 100 kHz, mehr bevorzugt von 20 Hz bis 20 kHz, über die Gewebeschichten und Messen des dabei fließenden frequenzabhängigen Wechselstroms.

Bekanntermaßen werden zur Ermittlung des elektrischen Impedanzspektrums Wechselspannungen beziehungsweise Wechselströme mit im wesentlichen sinusförmigen Verlauf und diskreten Einzelfrequenzen eingesetzt. Impedanzspektren werden dabei dann durch Einzelmessung bei verschiedenen diskreten Frequenzen, die, beispielsweise im Oktavsprung oder Dekadensprung, durchgestimmt werden, ermittelt. Die Erfindung sieht aber alternativ auch vor, Wechselspannungen beziehungsweise -ströme mit obertonhaltigem Verlauf, mit strukturiertem Rauschen und/oder transienten Impulsen einzusetzen, wodurch die Impedanz in mehreren Frequenzanteilen gleichzeitig gemessen und durch entsprechende Analyseverfahren, wie FFT und andere, registriert werden können. Durch die parallele, gleichzeitige Ermittlung der Impedanz bei mehreren Frequenzen kann die Messzeit verkürzt und/oder nachteilige Vorgänge an den Elektrodenmaterialien beziehungsweise Oberflächen, welche das Messergebnis beeinflussen können, vermieden oder reduziert werden.

In einer alternativen Ausgestaltung wird die elektrische Impedanz des biologischen Gewebes nur bei wenigen ausgewählten Frequenzen im Bereich von 1 Hz bis 100 kHz, beispielsweise 100 Hz und 1000 Hz, besonders bei einer einzigen Frequenz, beispielsweise 100 Hz, gemessen. In dieser Ausgestaltung kommt das erfindungsgemäße Analyseverfahren ohne den ansonsten notwendigen Kurvenfit der modellierten Spektren an das gemessene Modellspektrum aus. Vielmehr werde hier unter Vorgabe eines bestimmten ausgewählten Ersatzschaltbilds, beispielsweise Vorgabe bezüglich der elektrisch wirksamen Schichten sowie, alternativ oder zusätzlich, Vorgabe der Größe von elektrischem Serienwiderstand und konstantem Phasenelement (CPE) innerhalb der Messanordnung unmittelbar die biologischen Kenngrößen aus den Messdaten extrahiert.

Gegenstand der Erfindung ist auch ein Verfahren zur Charakterisierung der biologischen Wirkung eines Wirkstoffs auf ein biologisches mehrschichtiges Gewebe *in vitro,* enthaltend die Schritte:

A) erstes Ermitteln mindestens einer ersten biologischen Kenngröße $R_{Cell}$ und/oder $C_{Cell}(\omega)(N)$ nach dem Verfahren nach einem der vorstehenden Ansprüche bei dem biologischen Gewebe oder einer Gruppe biologischer Gewebe,

B) Inkontaktbringen dieses biologischen Gewebes oder dieser Gruppe biologischer Gewebe mit dem Wirkstoff, wobei mit dem Wirkstoff behandeltes Gewebe oder eine behandelte Gruppe solcher biologischer Gewebe erhalten wird, und

C) erneutes Ermitteln der mindestens einen biologischen Kenngröße $R_{Cell}$ und/oder $C_{Cell}(\omega)(N)$ nach dem Verfahren nach einem der vorstehenden Ansprüche bei dem behandelten biologischen Gewebe oder der behandelten Gruppe biologischer Gewebe,

D) Vergleichen der mindestens einen ersten ermittelten biologischen Kenngröße vor dem Inkontaktbringen mit der mindestens einen erneut ermittelten zweiten biologischen Kenngröße nach dem Inkontaktbringen, wobei eine Änderung der mindestens einen biologischen Kenngröße zwischen erster und erneuter Ermittlung eine biologische Wirkung dieses Wirkstoffs auf das biologische Gewebe anzeigt.

Gegenstand der Erfindung ist auch eine Vorrichtung zur Bestimmung biologischer Kenngrößen eines mehrschichtigen biologischen Gewebes *in vitro,* die speziell ausgebildet ist zur Durchführung des erfindungsgemäßen Mess- und Analyseverfahrens. Dazu enthält die Vorrichtung zumindest die Komponenten:

1) Messeinheit zur Messung eines Impedanzspektrums Z(ω) gemäß Schritt a) des Verfahrens nach einem der Ansprüche 1 bis 10, und

2) Recheneinheit, welche spezifisch programmiert ist zur automatischen Durchführung des erfindungsgemäßen Analyseverfahrens, wie dies besonders in den Schritten b) bis d) des hierin offenbarten Verfahrens charakterisiert ist. Die Recheneinheit kann dazu zumindest folgende Programmschritte ausführen:

- iteratives Anpassen von durch Modellierung elektrischer Kenngrößen erhaltenen n Modellspektren $Z_m(\omega)n$ an das gemessene Impedanzspektrum gemäß Schritt b) des Verfahrens,

- Bestimmen angepasster Modellspektren gemäß Schritt c) des Verfahrens, und

- Ermitteln mindestens einer Kenngröße des biologischen Gewebes gemäß Schritt d) des Verfahrens.

Bevorzugt enthält die Vorrichtung zusätzlich mindestens einen Bioreaktor zur Kultivierung von biologischem Gewebe *in vitro* mit Elektroden zur Spannungs-/Strombeaufschlagung und Messung der Impedanz über das kultivierte biologische Gewebe

Bevorzugt enthält der Bioreaktor eine Mehrzahl von Abteilungen zur parallelen getrennten Kultivierung mehrerer biologischer Gewebe *in vitro,* Elektroden, die jeder Abteilung individuell zugeordnet sind

Bevorzugt weist die Messeinheit dabei zusätzlich auf einen Multiplexer, der eine Mehrzahl von Elektroden des Bioreaktors mit der Messeinheit verschaltet, zur sequentiellen Messung der Impedanz in den mehreren parallel kultivierten biologischen Geweben.

Ein Bioreaktor stellt sterile Bedingungen bereit, in denen die biologischen Gewebe vermessen werden können. Bevorzugt ist ein Bioreaktor mit acht gleichartigen Messkammern, zur Bestimmung der Impedanzspektren von acht darin kultivierten biologischen Geweben. Der Bioreaktor bevorzugt aus einer Bioreaktor-Bodenplatte und einem Bioreaktor-Deckel in an sich bekannter Weise aus Kunststoffmaterial (besonders PEEK), aufgebaut. Ein solcher Bioreaktor ist beispielsweise aus DE 10 2009 022 345 A1 deren diesbezüglicher Offenbarungsgehalt in die Offenbarung der Erfindung mit einbezogen ist, bekannt. In der besonderen Ausgestaltung sieht der Bioreaktor vor, dass jedes biologische Gewebe auf einer Kulturmembran kultiviert wird, welche in einen separaten Rahmen oder Träger, ein sogenanntes Insert, gespannt ist. Dieses Insert wird jeweils in eine der mehreren in dem Bioreaktor vorhandenen getrennten Kammern eingesetzt. In besonderer Ausgestaltung sind in dem Bioreaktor Mittel und Maßnahmen vorgesehen, welches es ermöglichen, dass das biologische Gewebe in dem Insert und das Insert zentriert über den in dem Bioreaktor vorhandenen Elektroden positioniert wird. Bevorzugt ist vorgesehen, dass das biologische Gewebe in dem elektrischen Feld per Elektroden zentriert positioniert wird.

Die Elektroden des Bioreaktors sind bevorzugt Metallelektroden. In einer Variante sind die Elektroden an sich bekannte beschichtete Elektroden mit reduziertem Kontaktpotenzial, beispielsweise Ag/AgCl Elektroden. In einer alternativen und bevorzugten Variante sind die Elektroden aus korrosionsfestem Edelstahlmaterial gefertigt. Besonders ist dabei vorgesehen, die Elektrodenoberfläche glatt zu polieren, beispielsweise durch Elektropolieren. Dabei hat sich überraschend herausgestellt, dass durch die Reduktion der Rauigkeit der Oberfläche der elektropolierten Elektrode die Messung der elektrischen Impedanz verbessert.

In dem, bevorzugt zweiteilig in Basisteil und Deckelteil ausgebildeten, Bioreaktor ist bevorzugt vorgesehen, dass in der oberen Reaktorhälfte (Deckelteil) die Arbeitselektroden ausgebildet sind. Dabei ist bevorzugt, dass die oberen Arbeitselektroden, die vorzugsweise eine zylindrische Form aufweisen, in jeder Abteilung des Bioreaktors in ein becherförmiges Zellkulturinsert, eintauchen, sodass die Elektrodenoberfläche jeweils möglichst nahe an der Oberfläche des biologischen Gewebes zu liegen kommt. Bevorzugt ist die Arbeitselektrode in Form eines Stempels ausgebildet, der in die Reaktorkammer taucht. Während jede Abteilung des Bioreaktors eine separate Arbeitselektrode aufweist, dass die Gegenelektrode entweder ebenfalls für jede Abteilung getrennt ausgebildet sein oder aber in der Basis des Bioreaktors als durchgehende, mehrere Abteilungen übergreifende gemeinschaftliche Gegenelektrode ausgebildet sein. Bevorzugt ist vorgesehen, die Gegenelektrode in dem Boden des Bioreaktors fest zu integrieren. Diese ist beispielsweise als Plattenelektrode ausgeführt und umfasst bevorzugt den Boden mehrerer Abteilungen. Eine bevorzugte Ausgestaltung der Konstruktion des Bioreaktors beziehungsweise einer Abteilung davon ist in Figur 2 dargestellt.

Gegenstand der Erfindung ist auch die Verwendung der hierin beschriebenen Vorrichtung zum Zwecke der Bestimmung der biologischen Wirkung von Testsubstanzen auf *in vitro* kultiviertes mehrschichtiges biologisches Gewebe anhand der Änderung der ermittelten elektrischen Kenngrößen.

Gegenstand der Erfindung ist auch die Verwendung der hierin beschriebenen Vorrichtung zum Zwecke der Bestimmung der Reife und des Differenzierungsgrads von *in vitro* kultiviertem mehrschichtigem biologischem Gewebe anhand der ermittelten elektrischen Kenngrößen.

Gegenstand der Erfindung ist schließlich auch ein Computerprogrammprodukt, enthaltend die Instruktionen zur automatischen Durchführung des Verfahrens charakterisiert in den hierin beschriebenen Schritten b) bis d).

Die Erfindung wird durch die nachfolgenden Figuren und Ausführungsbeispielen näher beschrieben, ohne dass diese beschränkend zu verstehen wären.

Figur 1 zeigt, das der Datenanalyse der vorliegenden Erfindung zugrundeliegende technische Ersatzschaltbild eines biologischen Gewebes in Zusammenhang mit der elektrischen Impedanzmessung. Vom apikalen Pol 10 (Arbeitselektrode) des biologischen Gewebes bis zu dessen basalen Pol 60 (Gegenelektrode) enthält dessen Ersatzschaltbild ein apikales konstantes Phasenelement 20 einen Ohm'schen Serienwiderstand 30, und pro elektrisch wirksamer Schicht in dem biologischen Gewebe ein Netzwerk 40 aus kapazitiver Komponente 42 und Ohm'scher Komponente 44, welche parallel geschaltet sind, und dazu stromabwärts in Serie das basale konstante Phasenelement 50. Der elektrische Serienwiderstand 30 repräsentiert modellgemäß zusammengefasst sämtliche frequenzunabhängige Ohm'sche Serienwiderstände des Messaufbaus, inklusive Leitungswiderstände in den Zuleitungen und Elektroden ebenfalls ist der Widerstand der Elektrolytlösung (Kulturmedium) eine Komponente des elektrischen Serienwiderstands 30). Apikales und basales konstantes Phasenelement 20, 50 repräsentieren jeweils frequenzabhängige Phänomene im System, vor allem Vorgänge an den Grenzflächen der aktiven Elektrode beziehungsweise der Gegenelektrode. Diese sind gemäß dieses Modells von biologischen Effekten und Substanzwirkungen unabhängig; sie können als gemeinsames konstantes Phasenelement (CPE) zusammengefasst werden. Biologisch relevante Kenngrößen, welches erfindungsgemäß das biologische Gewebe charakterisieren und biologische Vorgänge anzeigen, sind die Ohm'sche Komponente 44 und die kapazitive Komponente 42 des Netzwerks 40 einer jeden elektrischen wirksamen Schicht $i$ des biologischen Gewebes. Weist das biologische Gewebe beispielsweise eine einzige elektrisch wirksame biologische Schicht auf ($n$ = 1) so enthält das Ersatzschaltbild ein einziges Netzwerk 40. Weist ein mehrschichtiges biologisches Gewebe zwei oder mehrere elektrisch wirksame Schichten auf, so weist das Ersatzschaltbild für jede elektrisch wirksame Schicht $i$ jeweils ein separates, zu den anderen Komponenten in Serie geschaltetes Netzwerk 40 auf.

Für jedes Netzwerk 40 können die zugehörigen Ohm'schen und kapazitiven Komponenten 42,44 jeweils unterschiedliche Werte umfassen. In einem vereinfachten alternativen erfindungsgemäßen Modell, sind die elektrischen Parameter von Ohm'scher und kapazitiver Komponente 42, 44 für jedes Netzwerk 40 gleich groß gesetzt. Für ein weiter vereinfachtes erfindungsgemäßes Netzwerk beträgt die Zahl $n=2$ und somit weist das Ersatzschaltbild genau zwei in Serie geschaltete Netzwerke 40 auf. In einer alternativen vereinfachten Variante der Erfindung weist das elektrische Ersatzschaltbild genau ein Netzwerk 40 auf ($n$ =1).

Für das erfindungsgemäße Ersatzschaltbild ist kennzeichnend, dass die in den Netzwerken 40 jeweils vorhandene kapazitive Komponente 42 als reale Kapazität mit realen Oberflächeneigenschaften modelliert ist. Die Idealität **N** ist dabei < 1.

Figur 2 zeigt einen schematischen Aufbau eines Bioreaktors oder einer Abteilung davon mit Basisteil 110 und Deckelteil 120, der mindestens eine Zellkulturkammer 130 bildet, worin ein Zellkulturinsert 140 eingehängt ist, welches die Zellkulturmembran 145 trägt. Auf der Zellkulturmembran 145 ist beispielhaft zur Veranschaulichung ein mehrschichtiges Gewebe 150 platziert dargestellt. In dem (hier beispielhaft dargestellten) Gewebe 150 ist eine apikale elektrisch wirksame Schicht 152 ausgebildet.

Das basale Kulturmedium 132 ist von dem apikalem Kulturmedium 134 elektrisch isoliert. In dem Basisteil 110 ist eine elektrische Gegenelektrode 115 eingesetzt. In dem Deckelteil 120 ist eine stempelförmige Arbeitselektrode 125 vorhanden, welche im zusammengesetzten Zustand des Bioreaktors in das Lumen der Kammer 130 und besonders direkt in das Zellkulturinsert 140 ragt. Die Impedanz wird zwischen Gegenelektrode 115 und Arbeitselektrode 125 in dem zwischen den Elektroden platzierten biologischen Gewebe 150 gemessen.

In einem Bioreaktor ist in dem Basisteil 110 mindestens eine Reaktorkammer 130 ausgebildet, worin jeweils das biologische Gewebe 150 kultiviert werden kann.

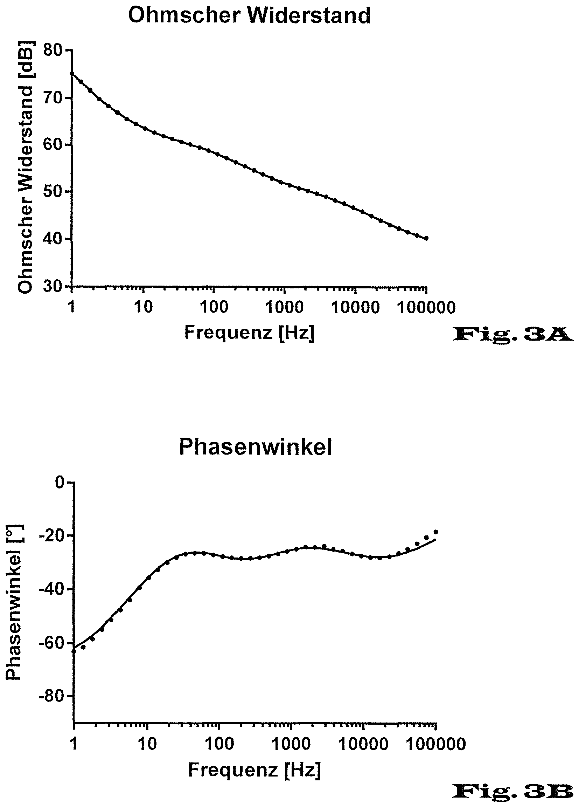

Figur 3 zeigt beispielhaft ein an einem mehrschichtigen biologischen Gewebe gemessenes elektrisches Impedanzspektrum, aufgezeichnet als Bode-Diagramme zu Amplitude (Betrag des komplexen Widerstands) und Phasenwinkel. Das gemessene Impedanzspektrum ist jeweils durch die konkreten Messpunkte repräsentiert. Überlagert ist das bestangepasste gemäß erfindungsgemäßem Ersatzschaltbilds modellierte Modellspektrum. Der Fit erfolgt nach der Methode der kleinsten Diffenenzenquadrate ("least mean square").

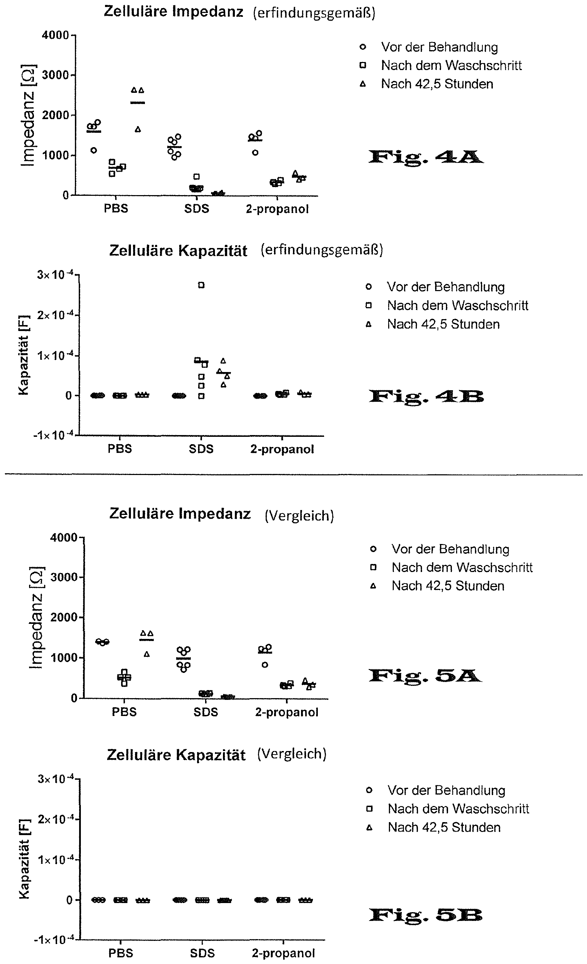

Figur 4 zeigt die mittels des erfindungsgemäßen Verfahrens ermittelten Kennwerte biologische Impedanz und biologische Kapazität der Epithelschicht mit *Stratum corneum* eines Hautäquivalents vor der Behandlung mit nicht irritativer Substanz (PBS), hoch irritativer Substanz (SDS) oder sub-irritativer Substanz (2-Propanol) für etwa 35 min, nach einem initialen Waschschritt sowie nach Erholung nach etwa 42,5 h. Figur 4A zeigt die zelluläre Impedanz, Figur 4B zeigt die Kapazität.

Figur 5 zeigt nach einem Vergleichsverfahren (*n* = 1, **N** = 1) ermittelte Kennwerte aus denselben gemessenen Impedanzspektren wie in Figur 4. Figur 5A zeigt die zelluläre Impedanz, Figur 5B zeigt die zelluläre Kapazität.

**Beispiel: Nachweis sub-irritativer Wirkungen von Substanzen in biologischen Geweben**

Mehrschichtige In-vitro-Hautäquivalente werden in an sich bekannter Weise aus isolierten Zellen, und zwar primäre Fibroblasten und Keratinozyten, rekonstituiert. Im Einzelnen wird in einem ersten Schritt der dermale Teil des Hautäquivalents aufgebaut, worin primäre Fibroblasten in eine Kollagenmatrix mit überwiegend Kollagentyp I durch Suspension integriert werden. In einem zweiten Schritt folgt das Überschichten der so gebildeten Dermis mit Keratinozyten, welche dann die epidermale Schicht bilden. Optional kann vor dem Aufbringen der Keratinozyten die gebildete Kollagenmatrix mit Fibronektin überschichtet werden, um eine Membran zu bilden. Der Aufbau des In-vitro-Hautäquivalents umfasst insgesamt etwa 21 Tage. In der letzten Kultivierungsphase befinden sich die mehreren Hautäquivalente in jeweils in an sich bekannten Zellkulturinserts in einem Bioreaktor, in dessen Basisteil eine planare Gegenelektrode eingesetzt ist. Im oberen Deckelteil befindet sich eine stempelförmige Arbeitselektrode, welche in das apikale Zellkulturmedium eingetaucht ist. Apikales Zellkulturmedium und basales Zellkulturmedium sind jeweils elektrisch voneinander isoliert und stehen nur über das *in vitro* Hautäquivalent miteinander in Verbindung.

Zur Messung der elektrischen Impedanz der einzelnen Hautäquivalente in dem Bioreaktor wird ein Wechselstrom mit wechselnder Frequenz mit einer Amplitude von etwa 3 mA zwischen apikaler Arbeitselektrode, welche den elektrischen Pluspol darstellt und basaler Gegenelektrode, welcher den elektrischen Minuspol darstellt, angelegt. Die Frequenz wird in 40 logarithmisch skalierten Stufen von 1 Hz bis 100 kHz variiert. Es werden sinusförmige Wechselströme mit diskreter Frequenz eingesetzt.

Figur 4 zeigt die so ermittelten Kenngrößen für die Behandlung mit drei bekannten Modellsubstanzen für irritative Wirkungen (gemäß CLP-Verordnung der Europäischen Union beziehungsweise des GHS-Standards):

1. Phosphatgepufferte Saline (PBS), welche beispielhaft für nicht irritative Substanzen steht,

2. 2-Propanol, welches beispielhaft für sub-irritative Substanzen steht,

3. Natriumdodecylsulfat 5 % (SDS), welches beispielhaft für hoch irritative und abrasive Substanzen steht.

Zur Untersuchung der Substanzwirkung auf das Hautäquivalent wird die Messung der elektrischen Impedanz jeweils vor der Behandlung durchgeführt (Kontrollmessung) und unmittelbar nach Inkontaktbringen des Hautäquivalents mit einer der Kontrollsubstanzen für etwa 35 min und anschließendem mehrfachem Waschen mit Kulturmedium (erste Messung). Eine weitere Messung erfolgt nach einer sogenannten Erholungsphase, etwa 42,5 Stunden nach dem ersten Inkontaktbringen mit der Kontrollsubstanz (zweite Messung).

Für jedes gemessene Impedanzspektrum werden die erfindungsgemäßen elektrischen Kenngrößen durch Fitten der erfindungsgemäß modellierten Modellspektren an die jeweils gemessenen Impedanzspektren ermittelt.

In einem Kontrollansatz werden dieselben gemessenen Impedanzspektren (Kontrolle, erste Messung, zweite Messung) zur Ermittlung elektrischer Kenngrößen an Modelle gefittet, welche stets nur eine einzige elektrisch wirksame Schicht (*n* = 1) vorsehen und wobei die das biologische Gewebes repräsentierende kapazitive Komponente durch einen idealen Kondensator ohne Berücksichtigung der Idealität **N** repräsentiert wird (**N** = 1). Figur 5A und 5B zeigt die so ermittelten Vergleichsdaten.

Aus dem Vergleich des erfindungsgemäßen Analyseverfahrens einem Vergleichsverfahren zeigt sich, dass insbesondere eine sub-irritative Wirkung, hier repräsentiert durch in Kontaktbringen mit der Substanz 2-propanol, mit dem Vergleichsverfahren nicht detektiert werden kann. Daneben erlaubt das erfindungsgemäße Analyseverfahren auch eine Quantifizierung der irritativen Wirkung von Substanzen, wohingegen das Vergleichsverfahren aufgrund seiner geringen Sensitivität irritativer und sub-irritativer Wirkungen von Substanzen keine hinreichend aussagekräftige Quantifizierung zulässt, da hier keine signifikante Dosis-Wirkung-Beziehung etabliert werden kann.

**Patentansprüche**

1. Verfahren zur Charakterisierung eines biologischen Gewebes *in vitro,* enthaltend die Schritte:

   a) Messen der elektrischen Impedanz des biologischen Gewebes in Abhängigkeit von der Frequenz, wobei ein Impedanzspektrum $Z(\omega)$ erhalten wird;
   b) Anpassen von n Modellspekten $Z_m(\omega)n$ an das gemessene Impedanzspektrum $Z(\omega)$, wobei jedes $Z_m(\omega)n$ durch eine Formel bestimmt ist, die ausgewählt ist aus der Formelschar (1):

$$Z_m(\omega)n = R_S + Z_{CPE}(\omega) + \text{Summe} \ (Z_{Cell}(\omega)i) \ \text{für } i = 1 \ \text{bis } n, \qquad (1)$$

wobei für jedes $i$

$Z_{Cell}(\omega)i$ die Impedanz einer elektrisch wirksamen Schicht $i$ in dem Gewebe ist, die einer Parallelschaltung der ohmschen Komponente $R_{Cell}i$ dieser Schicht und der realen kapazitiven Komponente $C_{Cell}(\omega)(Ni)i$ dieser Schicht gemäß Formel (2) entspricht:

$$Z_{Cell}(\omega)i = R_{Cell}i \ \| \ C_{Cell}(\omega)(Ni)i \qquad (2)$$

und wobei

$Ni$ die Idealität dieser realen kapazitiven Komponente $C_{Cell}(\omega)(Ni)i$ ist, die stets kleiner als 1 ist,

wobei für jedes $n$ die Modellspektren $Zm(\omega)n$ jeweils durch Variation von jeweils mindestens einer der impedanzbestimmenden Komponenten $R_{Cell}i$ und kapazitiver Komponente $C_{Cell}(\omega)(Ni)i$ jeder elektrisch wirksamen Schicht i an das in Schritt a) gemessene Impedanzspektrum $Z(\omega)$ angenähert werden;

c) Bestimmen für jedes Modellspektrum $Z_m(\omega)n$ das für jedes $n$ jeweils optimal an das gemessene Impedanzspektrum $Z(\omega)$ angepasste Modellspektrum, wobei das jeweils optimal angepasste Modell **dadurch gekennzeichnet ist, dass** es die jeweils kleinsten Residuen gegenüber dem gemessenen Impedanzspektrum aufweist; und

d) Ermitteln mindestens einer Kenngröße, ausgewählt aus ohmscher Komponente $R_{Cell}$ und kapazitiver Komponente, jedes optimal angepassten Modellspektrums als das biologische Gewebe charakterisierende Kenngröße.

2. Verfahren nach Anspruch 1, wobei die iterative Anpassung in Schritt b) nach der Methode der kleinsten Differenzenquadrate erfolgt.

3. Verfahren nach einem der vorstehenden Ansprüche, wobei in Schritt c) unter den $n$ gefundenen optimal angepassten Modellen $Z_m(\omega)n$, dasjenige als das bestangepasste Modell $Z_m(\omega)$ ausgewählt wird, welches über alle $n$ Modelle betrachtet, die kleinsten Abweichungen zu dem gemessenen Impedanzspektrum $Z(\omega)$ aufweist und in Schritt d) die mindestens eine Kenngröße aus diesem bestangepassten Modellspektrum $Z_m(\omega)$ ermittelt wird.

4. Verfahren nach Anspruch 3, wobei in Schritt d) für jede elektrisch wirksame Schicht $i$ des gefundenen bestangepassten Modellspektrums mindestens eine Kenngröße, ausgewählt aus ohmscher Komponente $R_{Cell}i$ und kapazitiver Komponente $C_{Cell}(\omega)(Ni')i$ als eine genau diese elektrisch wirksame Schicht $i$ des biologischen Gewebes charakterisierende Kenngröße getrennt ermittelt wird.

5. Verfahren nach einem der Ansprüche 3 oder 4, wobei in der Formel $Z_m(\omega)n$ des gefundenen bestangepassten Modellspektrums n die Zahl der in dem biologischen Gewebe aktuell vorherrschenden elektrisch wirksamen Schichten anzeigt.

6. Verfahren nach einem der vorstehenden Ansprüche, wobei $n = 1$ ist oder $n = 2$ ist.

7. Verfahren nach einem der vorstehenden Ansprüche, wobei in Schritt a) die Messung des Impedanzspektrums des biologischen Gewebes erfolgt durch Einprägen eines Wechselstroms mit wechselnden Frequenzanteilen im Bereich von 1 Hz bis 100 kHz und Messen der frequenzabhängigen Wechselspannung die über die Gewebeschichten hinweg abfällt.

8. Verfahren nach einem der vorstehenden Ansprüche, wobei in Schritt a) die Messung des Impedanzspektrums des biologischen Gewebes erfolgt durch Anlegen einer Wechselspannung mit wechselnden Frequenzanteilen im Bereich von 1 Hz bis 100 kHz über die Gewebeschichten und Messen des dabei fließenden frequenzabhängigen Wechselstroms.

9. Verfahren nach einem der vorstehenden Ansprüche, wobei das mehrschichtige biologische Gewebe ausgewählt ist aus:

- *de novo* aus isolierten Zellen des menschlichen oder tierischen Körpers und oder Zelllinien rekonstituiertem Gewebeäquivalent und

- explantiertem *ex vivo* Gewebe des menschlichen oder tierischen Körpers.

10. Verfahren zur Charakterisierung der Wirkung eines Wirkstoffs auf ein biologisches Gewebe *in vitro,* enthaltend die Schritte:

A) erstes Ermitteln mindestens einer ersten biologischen Kenngröße $R_{Cell}$ und/oder $C_{Cell}(\omega)(N)$ nach dem Verfahren nach einem der vorstehenden Ansprüche bei dem biologischen Gewebe oder einer Gruppe biologischer Gewebe,
B) Inkontaktbringen dieses biologischen Gewebes oder dieser Gruppe biologischer Gewebe mit dem Wirkstoff, wobei mit dem Wirkstoff behandeltes Gewebe oder eine behandelte Gruppe solcher biologischer Gewebe erhalten wird, und
C) erneutes Ermitteln der mindestens einen biologischen Kenngröße $R_{Cell}$ und/oder $C_{Cell}(\omega)(N)$ nach dem Verfahren nach einem der vorstehenden Ansprüche bei dem behandelten biologischen Gewebe oder der behandelten Gruppe biologischer Gewebe,
D) Vergleichen der mindestens einen ersten ermittelten biologischen Kenngröße vor dem Inkontaktbringen mit der mindestens einen erneut ermittelten zweiten biologischen Kenngröße nach dem Inkontaktbringen, wobei eine Änderung der mindestens einen biologischen Kenngröße zwischen erster und erneuter Ermittlung eine biologische Wirkung dieses Wirkstoffs auf das biologische Gewebe anzeigt.

11. Vorrichtung zur Bestimmung biologischer Kenngrößen eines biologischen Gewebes oder Gewebeäquivalents *in vitro,* enthaltend:

- Messeinheit zur Messung eines Impedanzspektrums $Z(\omega)$ gemäß Schritt a) des Verfahrens nach einem der Ansprüche 1 bis 9, und
- Recheneinheit, die programmiert ist zum:
- iterativen Anpassen von durch Modellierung elektrischer Kenngrößen erhaltenen n Modellspektren $Zm(\omega)n$ an das gemessene Impedanzspektrum gemäß Schritt b) des Verfahrens nach einem der Ansprüche 1 bis 9,
- Bestimmen angepasster Modellspektren gemäß Schritt c) des Verfahrens nach einem der Ansprüche 1 bis 9, und
- Ermitteln mindestens einer Kenngröße des biologischen Gewebes gemäß Schritt d) des Verfahrens nach einem der Ansprüche 1 bis 10.

12. Vorrichtung nach Anspruch 11, zusätzlich enthaltend:

- Bioreaktor zur Kultivierung von biologischem Gewebe *in vitro* mit Elektroden zur Spannungs-/Strombeaufschlagung und Messung der Impedanz über das kultivierte biologische Gewebe.

13. Vorrichtung nach Anspruch 12, wobei der Bioreaktor aufweist:

- Abteilungen zur parallelen, getrennten Kultivierung mehrerer biologischer Gewebe,
- Elektroden, die jeder Abteilung individuell zugeordnet sind.

14. Vorrichtung nach Anspruch 12 oder 13, wobei der Bioreaktor aufweist:

- Multiplexer, der eine Mehrzahl von Elektroden mit der Messeinheit verschaltet, zur sequentiellen Messung der Impedanzspektren in den mehreren parallel kultivierten biologischen Geweben.

15. Computerprogramm, enthaltend die Instruktionen zur automatischen Durchführung des Verfahrens der Schritte b) bis d) nach einem der Ansprüche 1 bis 9.

16. Verwendung der Vorrichtung nach einem der Ansprüche 11 bis 14 zur Bestimmung der biologischen Wirkung von Substanzen auf *in vitro* kultiviertes biologisches Gewebe anhand der Änderung der ermittelten elektrischen Kenngrößen.

17. Verwendung der Vorrichtung nach einem der Ansprüche 11 bis 14 zur Bestimmung der Reife und des Differenzierungsgrads von *in vitro* kultiviertem biologischem Gewebe anhand der ermittelten elektrischen Kenngrößen.

**Claims**

1. A method for characterizing a biological tissue *in vitro* containing the following steps:

   a) Measurement of the electrical impedance of the biological tissue as a function of frequency, wherein an impedance spectrum $Z(\omega)$ is obtained;
   b) Matching of n model spectra $Z_m(\omega)n$ to the measured impedance spectrum $Z(\omega)$, wherein each $Z_m(\omega)n$ is determined by a formula which is selected from the family of formulae (1):

$$Z_m(\omega)n = R_S + Z_{CPE}(\omega) + \text{Sum of } (Z_{Cell}(\omega)i) \text{ for } i = 1 \text{ to } n, \qquad (1)$$

   wherein for each $i$
   $Z_{Cell}(\omega)i$ is the impedance of an electrically active layer $i$ in the tissue which corresponds to a parallel circuit of the ohmic component $R_{Cell}i$ of this layer and the real capacitive component $C_{Cell}(\omega)(Ni)i$ of this layer according to Formula (2):

$$Z_{Cell}(\omega)i = R_{Cell}i \; \| \; C_{Cell}(\omega)(Ni)i \qquad (2)$$

   and wherein
   $Ni$ is the ideality of this real capacitive component $C_{Cell}(\omega)(Ni)i$ and is always less than 1;
   wherein, for each $n$, the model spectra $Zm(\omega)n$ are in each case approximated to the impedance spectrum $Z(\omega)$ measured in Step a) by variation of at least one of the impedance-determining components $R_{Cell}i$ and capacitive component $C_{Cell}(\omega)(Ni)i$ of each electrically active layer $i$ in each case.
   c) Determination for each model spectrum $Z_m(\omega)n$ the model spectrum which for each $n$ is optimally matched to the measured impedance spectrum $Z(\omega)$, wherein the optimally matched model in each case is **characterized in that** it has the smallest residues compared with the measured impedance spectrum in each case; and
   d) Calculation of at least one variable selected from ohmic component $R_{Cell}$ and real capacitive component of each optimally matched model spectrum as the variable which characterizes the biological tissue.

2. The method according to claim 1, wherein the iterative matching in Step b) is carried out using the method of least mean squares.

3. The method according to one of the preceding claims, wherein, in Step c), from the $n$ optimally matched models $Z_m(\omega)n$ found, the one which when viewed over all $n$ models has the smallest deviations from the impedance spectrum $Z(\omega)$ measured is chosen as the best matched model $Z(\omega)$, and in Step d), the at least one variable is calculated from this best matched model spectrum $Z(\omega)$.

4. The method according to claim 3, wherein, in Step d), for each electrically active layer $i$ of the best matched model spectrum found, at least one variable, selected from ohmic component $R_{Cell}i$ and capacitive component $C_{Cell}(\omega)(Ni)i$, is calculated separately as a variable which accurately characterizes this electrically active layer $i$ of the biological tissue.

5. The method according to one of claims 3 or 4, wherein, in the formula $Z_m(\omega)n$ of the best matched model spectrum found, $n$ indicates the number of electrically active layers actually prevailing in the biological tissue.

6. The method according to one of the preceding claims, wherein $n = 1$ or $n = 2$.

7. The method according to one of the preceding claims, wherein, in Step a), the impedance spectrum of the biological tissue is measured by injecting an alternating current with alternating frequency components in the range from 1 Hz to 100 kHz and measuring the frequency-dependent alternating voltage which drops across the tissue layers.

8. The method according to one of the preceding claims, wherein, in Step a), the impedance spectrum of the biological tissue is measured by applying an alternating voltage with alternating frequency components in the range from 1 Hz to 100 kHz across the tissue layers and measuring the flowing frequency-dependent alternating current as a result.

9. The method according to one of the preceding claims, wherein the multilayer biological tissue is selected from:

- tissue equivalent reconstituted *de novo* from isolated cells of the human or animal body and/or from cell lines and
- explanted *ex vivo* tissue of the human or animal body.

10. A method for characterizing the effect of an active substance on a biological tissue *in vitro,* containing the following steps:

A) Initial calculation of at least one first biological variable $R_{Cell}$ and/or $C_{Cell}(\omega)(N)$ according to the method according to one of the preceding claims for the biological tissue or a group of biological tissues,
B) Bringing this biological tissue or this group of biological tissues into contact with the active substance, wherein tissue treated with the active substance or a treated group of such biological tissues is obtained, and
C) Re-calculation of the at least one biological variable $R_{Cell}$ and/or $C_{Cell}(\omega)(N)$ according to the method according to one of the preceding claims for the treated biological tissue or the treated group of biological tissues,
D) Comparison of the at least one first calculated biological variable before bringing into contact with the at least one re-calculated second biological variable after bringing into contact, wherein a change in the at least one biological variable between first and repeated calculation indicates a biological effect of this active substance on the biological tissue.

11. A device for determining biological variables of a biological tissue or tissue equivalent *in vitro,* including:

- Measuring unit for measuring an impedance spectrum $Z(\omega)$ according to Step a) of the method according to one of claims 1 to 9, and
- Arithmetic unit, programmed for:
- Iterative matching of *n* model spectra $Zm(\omega)n$ obtained by modeling electrical variables to the measured impedance spectrum according to Step b) of the method according to one of claims 1 to 9,
- Determination of matched model spectra according to Step c) of the method according to one of claims 1 to 9, and
- Calculation of at least one variable of the biological tissue according to Step d) of the method according to one of claims 1 to 10.

12. The device according to claim 11, additionally including:

- A bioreactor for cultivating biological tissue *in vitro* with electrodes for applying voltage/current and measuring the impedance over the cultivated biological tissue.

13. The device according to claim 12, wherein the bioreactor has:

- Compartments for parallel separate cultivation of a plurality of biological tissues,
- Electrodes which are associated with each compartment individually.

14. The device according to claim 12 or 13, wherein the bioreactor has:

- A multiplexer which connects a plurality of electrodes to the measuring unit for sequential measurement of the impedance spectra in the plurality of parallel cultivated biological tissues.

15. A computer program including the instructions for automatically carrying out the method of Steps b) to d) according to one of claims 1 to 9.

16. The use of the device according to one of claims 11 to 14 for determining the biological effect of substances on *in vitro* cultivated biological tissue based on the change in the calculated electrical variables.

17. The use of the device according to one of claims 11 to 14 for determining the maturity and degree of differentiation of *in vitro* cultivated biological tissue based on the calculated electrical variables.

**Revendications**

1. Procédé de caractérisation d'un tissu biologique *in vitro*, contenant les étapes de :

a) mesure de l'impédance électrique du tissu biologique en fonction de la fréquence, dans lequel un spectre d'impédance Z(ω) est obtenu ;
b) adaptation de *n* spectres de modèle Z$_m$(ω)*n* au spectre d'impédance Z(ω) mesuré, dans lequel chaque Z$_m$(ω)*n* est déterminé par une formule qui est sélectionnée parmi le système de formules (1) :

$$Z_m(\omega)n = R_S + Z_{CPE}(\omega) + \text{Somme } (Z_{Cell}(\omega)i), \text{ pour } i = 1 \text{ à } n, \qquad (1)$$

dans laquelle pour chaque *i*
Z$_{Cell}$(ω)*i* est l'impédance d'une couche électriquement efficace *i* dans le tissu qui correspond à un montage en parallèle du composant ohmique R$_{Cell}$*i* de cette couche et du composant capacitif réel C$_{Cell}$((ω)(N*i*)*i* de cette couche selon la formule (2) :

$$Z_{Cell}(\omega)i = R_{Cell}i \parallel C_{Cell}(\omega)(Ni)i \qquad (2)$$

et dans laquelle
N*i* est l'idéalité de ce composant capacitif réel C$_{Cell}$((ω)(N*i*)*i* qui est toujours inférieure à 1,
dans lequel pour chaque *n,* les spectres de modèle Zm(ω)*n* s'approchent à chaque fois du spectre d'impédance Z(ω) mesuré à l'étape a) par variation à chaque fois d'au moins un des composants déterminant l'impédance R$_{Cell}$*i* et du composant capacitif C$_{Cell}$(ω)(N*i*)*i* de chaque couche électriquement efficace *i* ;
c) détermination pour chaque modèle de spectre Z$_m$(ω)*n* du spectre de modèle adapté pour chaque *n* à chaque fois de manière optimale au spectre d'impédance Z(ω) mesuré, dans lequel le modèle adapté à chaque fois de manière optimale est **caractérisé en ce qu'**il présente les résidus à chaque fois les plus petits par rapport au spectre d'impédance mesuré ; et
d) établissement d'au moins une grandeur caractéristique, sélectionnée parmi le composant ohmique R$_{Cell}$ et le composant capacitif, de chaque spectre de modèle adapté de manière optimale en tant que grandeur caractéristique caractérisant le tissu biologique.

2. Procédé selon la revendication 1, dans lequel l'adaptation itérative à l'étape b) s'effectue selon la méthode des moindres carrés des différences.

3. Procédé selon une des revendications précédentes, dans lequel à l'étape c) parmi les n modèles trouvés Z$_m$(ω)*n*, adaptés de manière optimale, celui qui, considéré sur tous les n modèles, présente les plus faibles écarts par rapport au spectre d'impédance Z(ω) mesuré est sélectionné en tant que modèle Zm(ω) le mieux adapté, et à l'étape d), l'au moins une grandeur caractéristique est établie à partir de ce spectre de modèle Z$_m$(ω) le mieux adapté.

4. Procédé selon la revendication 3, dans lequel à l'étape d) pour chaque couche électriquement efficace *i* du spectre de modèle trouvé le mieux adapté, au moins une grandeur caractéristique, sélectionnée parmi le composant ohmique R$_{Cell}$*i* et le composant capacitif C$_{Cell}$((ω)(N*i*)*i*, est établie séparément en tant que grandeur caractéristique caractérisant précisément cette couche électriquement efficace *i* du tissu biologique.

5. Procédé selon une des revendications 3 ou 4, dans lequel dans la formule Z$_m$(ω)*n* du spectre de modèle trouvé le mieux adapté, *n* indique le nombre de couches électriquement efficaces, prédominant actuellement dans le tissu biologique.

6. Procédé selon une des revendications précédentes, dans lequel *n* est = 1 ou *n* est = 2.

7. Procédé selon une des revendications précédentes, dans lequel à l'étape a), la mesure du spectre d'impédance du tissu biologique s'effectue en conférant à un courant alternatif des portions de fréquence alternatives dans la plage de 1 Hz à 100 kHz et mesurant la tension alternative dépendant de la fréquence qui diminue sur les couches de tissu.

8. Procédé selon une des revendications précédentes, dans lequel à l'étape a), la mesure du spectre d'impédance du tissu biologique s'effectue en appliquant une tension alternative avec des portions de fréquence alternatives dans la plage de 1 Hz à 100 kHz sur les couches de tissu et mesurant le courant alternatif dépendant de la fréquence qui s'écoule ce faisant.

9. Procédé selon une des revendications précédentes, dans lequel le tissu biologique multicouches est sélectionné

EP 3 183 563 B1

parmi :

- de l'équivalent de tissu *de novo* reconstitué à partir de cellules isolées du corps humain ou animal et ou de lignées cellulaires et
- du tissu *ex vivo* explanté du corps humain ou animal.

10. Procédé de caractérisation de l'action d'une substance active sur un tissu biologique *in vitro*, contenant les étapes de :

A) premier établissement d'au moins une première grandeur caractéristique biologique $R_{Cell}$ et/ou $C_{Cell}(\omega)(N)$ selon le procédé selon une des revendications précédentes dans le cas du tissu biologique ou d'un groupe de tissus biologiques,
B) mise en contact de ce tissu biologique ou de ce groupe de tissus biologiques avec la substance active, dans lequel du tissu traité avec la substance active ou un groupe traité de tels tissus biologiques est obtenu, et
C) nouvel établissement de l'au moins une grandeur caractéristique biologique $R_{Cell}$ et/ou $C_{Cell}(\omega)(N)$ selon le procédé selon une des revendications précédentes dans le cas du tissu biologique traité ou du groupe traité de tissus biologiques,
D) comparaison de l'au moins une première grandeur caractéristique biologique établie avant la mise en contact à l'au moins une seconde grandeur caractéristique biologique nouvellement établie après la mise en contact, dans laquelle une modification de l'au moins une grandeur caractéristique biologique entre le premier et le nouvel établissement indique une action biologique de cette substance active sur le tissu biologique.

11. Dispositif de détermination de grandeurs caractéristiques biologiques d'un tissu ou équivalent de tissu biologique *in vitro*, contenant :

- un module de mesure pour la mesure d'un spectre d'impédance $Z(\omega)$ selon l'étape a) du procédé selon une des revendications 1 à 9, et
- un compteur, qui est programmée pour :
- l'adaptation itérative de $n$ spectres de modèle $Zm(\omega)n$ obtenus par modélisation de grandeurs caractéristiques électriques au spectre d'impédance mesuré selon l'étape b) du procédé selon une des revendications 1 à 9,
- la détermination de spectres de modèle adaptés selon l'étape c) du procédé selon une des revendications 1 à 9, et
- l'établissement d'au moins une grandeur caractéristique du tissu biologique selon l'étape d) du procédé selon une des revendications 1 à 10.

12. Dispositif selon la revendication 11, contenant en outre :

- un bioréacteur pour la culture de tissu biologique *in vitro* avec des électrodes pour la sollicitation en tension/courant et mesure de l'impédance sur le tissu biologique cultivé.

13. Dispositif selon la revendication 12, dans lequel le bioréacteur présente :

- des cases pour la culture parallèle séparée de plusieurs tissus biologiques,
- des électrodes qui sont associées individuellement à chaque case.

14. Dispositif selon la revendication 12 ou 13, dans lequel le bioréacteur présente :

- un multiplexeur, qui interconnecte une pluralité d'électrodes avec le module de mesure, pour la mesure séquentielle des spectres d'impédance dans les multiples tissus biologiques cultivés en parallèle.

15. Programme informatique, contenant les instructions pour la réalisation automatique du procédé des étapes b) à d) selon une des revendications 1 à 9.

16. Utilisation du dispositif selon une des revendications 11 à 14 pour la détermination de l'action biologique de substances sur du tissu biologique cultivé *in vitro* à l'aide de la modification des grandeurs caractéristiques électriques établies.

17. Utilisation du dispositif selon une des revendications 11 à 14 pour la détermination de la maturité et du degré de différenciation de tissu biologique cultivé *in vitro* à l'aide des grandeurs caractéristiques électriques établies.

**Fig. 1**

**Fig. 2**

## Ohmscher Widerstand

**Fig. 3A**

## Phasenwinkel

**Fig. 3B**

## Zelluläre Impedanz (erfindungsgemäß)

o Vor der Behandlung
□ Nach dem Waschschritt
Δ Nach 42,5 Stunden

**Fig. 4A**

## Zelluläre Kapazität (erfindungsgemäß)

o Vor der Behandlung
□ Nach dem Waschschritt
Δ Nach 42,5 Stunden

**Fig. 4B**

## Zelluläre Impedanz (Vergleich)

o Vor der Behandlung
□ Nach dem Waschschritt
Δ Nach 42,5 Stunden

**Fig. 5A**

## Zelluläre Kapazität (Vergleich)

o Vor der Behandlung
□ Nach dem Waschschritt
Δ Nach 42,5 Stunden

**Fig. 5B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10062623 A1
- DE 102010023156 A1
- DE 102009022345 A1